# EUROPEAN PATENT APPLICATION

(11) **EP 1 895 003 A1**
(43) Date of publication of application: **05.03.2008**
(21) Application number: 07023852.2
(22) Date of filing: 07.10.1999
(51) Int. Cl.: C12N 15/00, C12N 7/00, A61K 48/00, C12N 15/86

(54) **Cells expressing recombinase**

(30) Priority: 12.10.1998 JP 28978598
(62) Divisional of application: 99970420.8
(71) Applicant: Dainippon Sumitomo Pharma Co., Ltd., Osaka 541-8524 (JP)
(72) Inventor: Saito, Izumu, Shibuya-ku Tokyo 151-0053 (JP); Kanegae, Yumi, Tokyo 141-0031 (JP)
(74) Representative: Duckworth, Timothy John

(57) **Abstract**

The present invention provides DNA having a modified nucleotide sequence wherein the translation efficiency of the FLP protein in animal cells including human cells has been enhanced by changing a codon ratio preferably used in yeast to a codon ratio preferably used in humans by replacing a codon encoding an amino acid of the FLP protein in a nucleotide sequence encoding a yeast-derived FLP.

SEQUENCE LISTING

<110> Sumitomo Pharmaceuticals Co., Ltd.

<120> Cells Expressing Recombinase

<130> E4831-00

<150> JP 10-289785
<151> 1998-10-12

<160> 4

<210> 1
<211> 54
<212> DNA
<213> Artificial Sequence

<220>
<223> FLP recognition sequence

<400> 1
aaattccgga gaagttccta ttctctagaa agtataggaa cttcgacgtc attt          54

<210> 2
<211> 27
<212> DNA
<213> Artificial Sequence

<220>
<223> Polylinker

<400> 2
aaattgaatt cgagctcggt acccggg 27

<210> 3
<211> 27
<212> DNA
<213> Artificial Sequence

<220>
<223> Sense Strand of Adaptor

<400> 3
agcttctgca gcagaccgtg catcatg 27

<210> 4
<211> 19
<212> DNA
<213> Artificial Sequence

<220>
<223> Antisense Strand of Adaptor

<400> 4
atgcacggtc tgctgcaga 19

<210> 5

<211> 1285

<212> DNA

<213> Human

<400> 5

cagaccgtgc atcatgagcc agtttggcat cctgtgcaag acaccaccta aggtgctggt 60

gcgccagttc gtggagaggt ttgagaggcc ctctggagag aagattgcct cctgtgcagc 120

tgagctgacc tacctgtgct ggatgatcac ccacaacggc acagccatca agagggccac 180

ctttatgagc tacaacacca tcattagcaa ctccctgagc ttcgacattg tgaacaagtc 240

cctccagttt aaatacaaga cccagaaggc cacaatcctg gaggcctccc tgaagaaatt 300

gattcctgct tgggagttca ccatcatccc ctacaatggc cagaagcacc agtctgatat 360

cactgatatt gtgagcagtc tgcaactcca gttcgagtcc tctgaggaag ctgacaaggg 420

caacagccac agcaagaaga tgctgaaggc cctgctcagt gagggagaaa gcatctggga 480

gatcactgag aagatcctga actcctttga gtacacttcc agattcacca agaccaagac 540

cttgtaccag ttcctgttcc tggccacctt catcaactgt ggcaggttca gcgacatcaa 600

gaatgtggat cccaaatcct ttaaactggt ccagaacaag tacctgggag tgatcatcca 660

gtgcctggtg acagagacca agacctctgt gagcaggcac atctacttct tctctgccag 720

gggcaggatt gatccactgg tgtacctgga tgagttcctg aggaactctg agccagtgct 780

gaagcgggtg aacaggaccg gcaactcttc cagcaacaag caggagtacc agctgctcaa 840

ggacaacctg gtgaggtcct acaacaaagc tttgaagaaa aatgccccct acccaatctt 900

tgccatcaag aatggcccta agtcccacat tggcagacac ctgatgacct ccttcctgtc 960

catgaagggc ctgacagagc tgaccaatgt tgtgggcaac tggagcgata agcgggcctc 1020

tgccgtggcc agaacaacct atactcacca gatcacagca atccctgatc actacttcgc 1080

actggtgtct cggtactatg catatgatcc catctccaag gagatgattg cattgaagga 1140

tgagaccaac ccaattgagg agtggcagca cattgagcag ctgaagggta gtgccgaggg 1200

cagcattcgc taccctgcct ggaatgggat catttcccag gaggtgctgg actacctgtc 1260

ttcctacatc aacagacgca tctga 1285

## Description

### TECHNICAL FIELD

The present invention relates to recombinase-expressing cells and methods of constructing recombinant viral vectors using such cells.

### BACKGROUND ART

As viral vectors for introducing genes into animal cells or for gene therapy, there have been used retroviruses, adenoviruses, adeno-associated viruses, herpesviruses, and the like. When these viral vectors are used in gene therapy, they are desired to be vectors of a structure that minimizes the expression of proteins encoded by the viruses for safety reasons.

In retroviruses, virus-producing cells have been established that supply all the proteins required for the viral growth. On the other hand, in adenovirus vectors and herpesvirus vectors, virus-producing cells have not been established that supply all the proteins required for the viral growth because of the presence of too many types of proteins that are encoded by viruses and of the problem of possible cytotoxicity by the proteins. As an alternative method, a method that employs a helper virus is used in the construction of these viruses. This method intends to supply viral proteins required for viral growth from the helper virus to the vector virus which otherwise cannot propagate by itself since part or all of the genes essential for the growth have been eliminated therefrom, thereby allowing said vector to propagate together with the helper virus. In this method of using a helper virus, adenovirus vectors (Mitani et al., Proc. Natl. Acad. Sci. 92: 3854-3858, 1995) and herpesvirus vectors (Banerjee et al., Nature Medicine, 1: 1303-1308, 1995) have been constructed.

One of the problems associated with the construction of viral vectors using a helper virus is how to reduce the amount of the helper virus relative to that of the viral vector of interest. To that end, in adenovirus vectors, various attempts have been made such as an attempts to reduce the packaging efficiency of the helper virus DNA into virus particles (virions) using a helper virus derived from a packaging-sequence mutant thereby lowering the growth rate of the helper virus (Kochanek et al., Proc. Natl. Acad. Sci. 93: 5731-5736, 1996), or an attempt to eliminate the packaging sequence of a helper virus which is inserted of loxP sequence, a recognition sequence of recombinase Cre, into both sites flanking the packaging sequence, by infecting this helper virus to a recombinase Cre-expressing cell, (Parks et al., Proc. Natl. Acad. Sci. 93: 13565-13570, 1996), and the like.

In the latter method in particular, recombinase Cre-expressing cells are important, and consequently several cell lines that permanently express Cre have been reported (Parks et al., Proc. Natl. Acad. Sci. 93: 13565-13570, 1996; Chen et al., Somat. Cell Mol. Genet. 22: 477-488, 1996; Lieber et al., J. Virol. 70: 8944-8960, 1996). However, although cell lines that permanently express Cre can be established, it is considered difficult to establish stable cell lines that express Cre in large quantities under the control of a high-level expression promoter since recombinase Cre has cytotoxicity on animal cells (Lieber et al., J. Virol. 70: 8944-8960, 1996).

### DISCLOSURE OF THE INVENTION

It is an object of the present invention to provide a cell that expresses Cre at high level for use in the construction of various viral vectors. It is another object of the present invention to provide a more efficient method of constructing a recombinant viral vector using such a cell.

In the case of establishing cell lines that permanently express cytotoxic proteins including the Cre protein; in general, even though cells are transformed by the plasmid that has inserted the gene of said protein downstream of a high-level expression promoter, it is difficult to obtain cell lines that stably express said protein at high level, in most cases, cell lines that express the protein at a level in which the toxicity of said protein can be tolerated by the cell are only obtained.

On the other hand, promoters are known that induce the expression of proteins triggered by a drug, etc. However, it is known that the activity of such inducible promoters is generally lower than that of strong promoters that permanently express proteins.

Thus, as a means to obtain a stable cell line that expresses the Cre protein from a strong promoter, the inventors of the present invention have inserted a recognition sequence of recombinase FLP and a stuffer DNA in between the promoter and the Cre gene, and thereby have successfully controlled the expression of the Cre protein. Since it expresses little Cre protein in the absence of recombinase FLP, this cell line can avoid the cytotoxicity of Cre, and this cell line can express high levels of the Cre protein from a strong promoter in the presence of FLP.

Thus, the gist of the present invention relates to:
(1) a cell that expresses recombinase Cre in the presence of recombinase FLP in a FLP-dependent manner,
(2) a method of allowing the cell described in the above (1) to express recombinase Cre by introducing recombinase FLP into said cell,
(3) a method of producing a recombinant viral vector which comprises using a cell that expresses recombinase Cre in the presence of recombinase FLP in a FLP-dependent manner,
(4) a method of producing a recombinant adenovirus vector using the method described in said (2) and (3), and
(5) DNA having a modified nucleotide sequence in which the translation efficiency of the FLP protein in animal cells including human cells has been enhanced by changing a codon ratio preferably used in yeast to a codon ratio preferably used in humans by replacing a codon encoding an amino acid of the FLP protein in a nucleotide sequence encoding a yeast-derived FLP.

### BRIEF EXPLANATION OF DRAWINGS

Figure 1 is a schematic diagram showing the structure of plasmid pCALNLZ (A), plasmid pUFNF (B), and plasmid pCALNL5 (C). In the figure, CA Pro represents the CAG promoter, Neo^{R} represents the neomycin resistant gene, SpA represents the SV40 poly(A) sequence, and GpA represents the β-globin poly(A) sequence. The shaded area represents the loxP sequence, and the dark area represents the FRT sequence. Ap^{R} represents the ampicillin resistant gene, and ori represents the origin of replication. X represents a restriction enzyme XhoI site, and M represents a restriction enzyme MluI site.

Figure 2 is a schematic diagram showing the structure of plasmid pCAFNFNCre. NCre represents the Cre gene having a nuclear localization signal.

Figure 3 is a schematic diagram showing the structure of plasmid pxCAFLP (A), plasmid pxCAwt (B), and plasmid pxCALNLZ (C). In the figure, the-solid line area represents the adenovirus genome (about 0.4 kb).

Figure 4 shows an example of the nucleotide sequence of humanized FLP. In the figure, numerical values in the left column indicate base numbers from the origin of translation which was set number 1. Lowercases indicate bases substituted in the yeast sequence, and the underlined part indicates a site in which the amino acid sequence was replaced.

### BEST MODE FOR CARRYING OUT THE INVENTION

In accordance with the present invention, "recombinase FLP" is an enzyme that is encoded by 2 micron DNA of yeast (Saccharomyces cereviceae) and that performs site-specific recombination between two FLP recognition sequences (FRTs) (Babineau et al., J. Biol. Chem. 260: 12313-12319, 1985). FLP can excise DNA sequence flanked by two FRTs in the same direction. FRT is a nucleotide sequence comprising 34 bp (Jayaram et al., Proc. Natl. Acad. Sci. 82: 5874-5879, 1985). In accordance with the present invention, "a recognition sequence of FLP" is not specifically limited as long as it is a nucleotide sequence containing FRT.

In accordance with the present invention, "express recombinase Cre in the presence of recombinase FLP in a FLP-dependent manner" means that a cell containing DNA, in the genome thereof, constructed in such a way that the cell cannot express recombinase Cre in the absence of recombinase FLP begins to express recombinase Cre by excising, in the presence of recombinase FLP, DNA sequence flanked by two FRTs in the same direction.

In accordance with the present invention, "recombinase Cre" is a specific DNA recombinase encoded by an E. coli bacteriophage P1 and uses the loxP sequence (Abremski et al., J. Biol. Chem. 1509-1514, 1984; and Hoess et al., Proc. Natl. Acad. Sci. 81: 1026-1029, 1984) in the bacteriophage P1 as the substrate. Recombinase Cre recognizes the loxP sequence and conducts total processes including clevage of DNA, exchanging and binding strands with this sequence. Thus, the loxP sequence is a recognition sequence of recombinase Cre.

The recombinase Cre gene can be excised with a suitable restriction enzyme from a plasmid (for example pUCCre (Japanese Unexamined Patent Publication (Kokai) No.8-84589) in-which a portion encoding the recombinase gene of bacteriophage P1 DNA was amplified using, for example, a polymerase chain reaction (PCR) and cloned into said plasmid, and then can be used.

In accordance with the present invention, preferably a nuclear localization signal is connected to the 5'-end or 3'-end of the recombinase Cre gene sequence. This is because recombinase Cre synthesized in the cytoplasm needs to transport into the nucleus for it to act effectively on its target recognition sequence, DNA having the loxP sequence, which transport is promoted by the nuclear localization signal (Daniel Kalderon et al., Cell 39: 499-509, 1984). The Cre gene having the nuclear localization signal can be obtained from plasmid pSRNCre (Kanegae Y. et al., Nucleic Acids Res. 23: 3816-3821, 1995), and the like.

The above constructed DNA present in the genome of the cell of the present invention contains, specifically, a promoter, a recognition sequence of recombinase FLP, a stuffer sequence, a recognition sequence of recombinase FLP, and the recombinase Cre gene in this order from upstream.

As the above promoter, there can be mentioned, but not limited to, the SRα promoter (Molecular and Cellular Biology, 8: 466-472, 1991), the EF-1α promoter (Gene 91: 217-223, 1990), the CMV promoter, etc. as long as it functions in mammalian cells.

In the present invention, however, the CAG promoter is preferably used. This is a hybrid promoter (the CAG promoter) comprising the cytomegalovirus enhancer, the chicken β-actin promoter, the splicing acceptor and poly(A) sequence of rabbit β-globin, and is disclosed as a high-level expression promoter in Japanese Unexamined Patent Publication (Kokai) No. 3-168087. It can be prepared from pCAGGS (Japanese Unexamined Patent Publication (Kokai) No. 3-168087, page 13 line 20 to page 20 line 14, and page 22 line 1 to page 25 line 6) disclosed in the publication by excising with restriction enzymes SalI and HindIII and can be used for the present invention. Alternatively, it can be excised with commercially available suitable restriction enzymes and can be used.

As a recognition sequence of recombinase FLP, any nucleotide sequence containing FRT may be used as described above. Said sequence may be synthesized using a DNA synthesizer.

The above stuffer sequence represents a nucleotide sequence flanked by two recognition sequences of recombinase FLP in the same direction, and a nucleotide sequence that is excised in a circular form in the presence of FLP.

The stuffer sequence may be any sequence as long as it prevents the expression of the Cre gene, but in order to confirm the integration of the DNA of interest after transfecting the cell, it preferably contains a maker gene such as a drug resistant gene, more preferably a neomycin resistant gene that is preferably used in selection of mammalian cells. The stuffer sequence preferably contains a poly(A) sequence downstream of the drug resistant gene so that the drug resistant gene may be efficiently expressed and the recombinase Cre gene located downstream thereof may not be expressed. As the poly(A) sequence, there can be mentioned, but not limited to, a poly(A) sequence derived from SV40, the poly(A) sequence of rabbit β-globin, and the like. These drug resistant genes and poly(A) sequences may be commercially available.

As the poly(A) sequence used downstream of the recombinase Cre gene, there may be used, but not limited to, one derived from rabbit β-globin.

As the cell for use in the preparation of the cell of the present invention, any cell suitable for the growth of viral vector of interest may be used without limitation.

When the cells of the present invention are used for constructing an adenovirus vector, it is preferred to use a cell that expresses the adenovirus E1A gene. Cells that express the E1A gene can be constructed by a conventional method (Imler et al., Gene Ther. 3: 75-84, 1996), and the like, it is more preferred to use a human fetus kidney-derived cell line 293 cells (ATCC CRL1573). However, said cells need not be expressing only the E1A gene, and may be expressing other adenovirus genes or other genes.

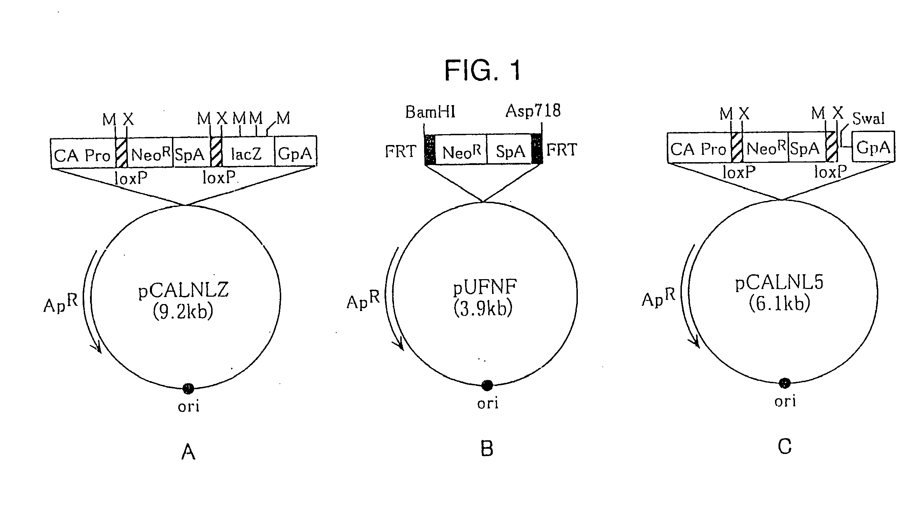

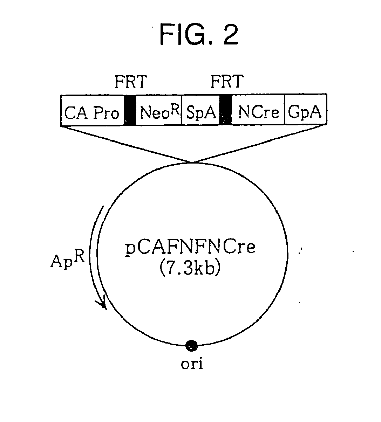

Now, by way of example, a preparation method of cells that express recombinase Cre in the presence of recombinase FLP in a FLP-dependent manner is explained.
[1] A plasmid is constructed that contains the 34 bp FRT sequence, two 54 bp DNAs (SEQ ID NO: 1), in the same direction, synthesized for introducing a restriction enzyme SwaI site therein, and a SwaI site in between the two FRT sequences. In the present invention, a plasmid that has introduced a SwaI site is preferably used hereinbelow.
[2] From plasmid pCALNLZ (Y. Kanegae et al., Gene 181: 207-212, 1996, Figure 1A), a fragment containing the neomycin resistant gene and the SV40 poly(A) sequence is prepared and then is inserted into the SwaI site of the plasmid obtained in the above [1] to obtain plasmid pUFNF (Figure 1B) in which said fragment is flanked by FRT sequences on both ends.
[3] A synthetic 27-base polylinker (SEQ ID NO: 2) is inserted into a SwaI site of plasmid pCALNLw (Y. Kanegae et al., Gene 181: 207-212, 1996) which is a source of the CAG promoter and the rabbit b-globin poly(A) sequence to obtain plasmid pCALNL5 (Figure 1C). From pUFNF obtained in the above [2], a fragment containing the FRT sequence/neomycin resistant gene/SV40 poly(A) sequence/FRT sequence is prepared and then is ligated to a fragment containing the above pCALNL5-derived CAG promoter to obtain plasmid pCAFNF5. This plasmid has a structure in which each of the two loxP sequences of pCALNL5 has been replaced with the FRT sequence.
[4] From plasmid pSRNCre (Y. Kanegae et al., Nucleic Acids Res. 23: 3816-3821, 1995), a fragment containing the nuclear localization signal added Cre gene (NCre) is prepared, which is inserted into the SwaI site of pCAFNF5 obtained in the above [3] to obtain plasmid pCAFNFNCre (Figure 2).
[5] After 293 cells are transfected (calcium phosphate coprecipitation method) with pCAFNFNCre obtained in the above [4], G418 (a neomycin derivative) resistant cells are cloned to obtain the cells (293FNCre cells) of the present invention.

The cells of the present invention such as the 293FNCre cells thus obtained can express recombinase Cre at a high level by introducing the recombinase FLP gene or the FLP protein into said cells. As a method of introducing the recombinase FLP gene, there can be mentioned a method of directly introducing plasmid by transfection, a method of using a viral vector, a liposome method, and the like. For cells suitable for adenovirus growth such as the 293FNCre cells, the FLP gene is preferably introduced using an adenovirus vector.

The cells of the present invention such as the 293FNCre cells can be used for the construction of viral vectors that employ helper viruses. By way of example, the construction of a recombinant adenovirus vector using the 293FNCre cells is specifically described hereinbelow.

A recombinant adenovirus vector (virus of interest) that only contains the inverted terminal repeat (ITR) and a packaging sequence of adenovirus and in which all other adenovirus genomes have been replaced with foreign genes cannot grow by itself, and thus it is allowed to grow in the presence of a helper virus. At this time, an attempt has been made: that is, in order to suppress the growth of the helper virus, the loxP sequence is inserted into both ends of the packaging sequence of the helper virus, and then Cre-expressing 293 cells are infected or transfected with this helper virus and the virus of interest or a plasmid containing the DNA of the virus of interest, so that the packaging sequence of the helper virus is deleted, its growth is suppressed, and the ratio of the virus of interest is enhanced (Parks et al., Proc. Natl. Acad. Sci. 93: 13565-13570, 1996). In this case, the more the amount of Cre expressed, more efficiently the packaging sequence of the helper virus is excised, with a result that the ratio of the virus of interest becomes higher.

The 293FNCre cells of the present invention, when used with the FLP-expressing recombinant adenovirus, can replace the Cre-expressing 293 cells, resulting in the expression of Cre in greater amounts than the 293 cells that permanently express Cre. Thus, it can excise the packaging sequence of the helper virus more efficiently than the Cre-expressing 293 cells so that the ratio of the virus of interest can be enhanced. At this time, by inserting in advance loxP sequences into both ends of the packaging sequence of the FLP-expressing adenovirus itself, the adenovirus can not only supply FLP but act as a helper virus.

Cells such as the 293FNCre cells of the present invention can be used not only for the construction of recombinant adenovirus vectors, but for the construction of adeno-associated viral (AAV) vectors. The following is an example of the construction of an AAV vector using the 293FNCre cells.

For the construction of an AAV vector, adenovirus as a helper virus must be infected to cells such as the 293 cells together with an AAV vector plasmid (Berns et al., Adv. Virus. Res., 32: 243-306, 1987). Since the adenovirus used as a helper virus must be removed from the AAV vector by a procedure such as heat inactivation after the formation of the AAV vector, it is preferred that the helper virus per se does not grow.

Using the 293FNCre cell of the present invention as an AAV-producing cell, and using the FLP-expressing adenovirus into which the loxP sequence has been inserted on both ends of the packaging sequence as a helper virus, the helper virus supplies proteins needed for the growth of AAV but the helper virus per se is not packaged into the infected particles, and thereby the AAV vector can be efficiently produced.

Furthermore, the cells of the present invention can also be used for the construction of other viruses that employ helper viruses. For example, a herpesvirus vector may be mentioned. While cells suitable for the growth of herpesvirus are transformed in a similar manner as for the 293FNCre cells so that Cre can be expressed in a FLP-dependent manner, the loxP sequence is inserted on both ends of the packaging sequence in the helper virus. Cells that express Cre in a FLP-dependent manner are transfected with a vector plasmid having the gene of interest, and that cells are also infected with the helper virus and allow the cells to express FLP by some means or other, the helper virus cannot grow so that the packaging sequence is eliminated and the vector virus of interest can be obtained. As an example of allowing the cells to express the FLP protein, there can be mentioned a method of inserting an expression unit of FLP into a helper virus.

Although recombinase FLP is an enzyme that performs DNA recombination in a similar manner to Cre, the possibility has been indicated that the FLP protein cannot be fully produced at an ordinary temperature (37° C) for use in the culture of animal cells even if the FLP gene is inserted into cells including humans. A report by Nakano et al. indicated the possibility that even if the FLP gene ligated downstream of the above CAG promoter which is a high-level expression promoter is inserted into an adenovirus vector which is also a high-level expression vector followed by the infection of said vector to a cultured animal cell, the FLP protein may not be fully produced (presentation No. 2463 at the 1998 Japan Cancer Society Meeting). Since the promoter used for the expression of the FLP gene is the CAG promoter which is a high-level expression promoter, the inadequate production of the FLP protein may be attributed that not the transcription step but the translation step is a rate- limiting step. In order to realize the full functioning of the FLP protein that was expressed by introducing FLP gene into an animal cell, it is essential to increase the amount of the expression of FLP protein in the animal cell.

Thus, the inventors of the present invention contrived to enhance the translation efficiency of the FLP protein in animal cells including human cells by changing the codon ratio of the codon of FLP protein derived from a yeast preferably used in yeast to the codon ratio preferably used in humans. Thus, we have obtained DNA sequence having modified nucleotide sequences in which the translation efficiency of the FLP protein in animal cells including human cells has been enhanced by changing a codon ratio preferably used in yeast to a codon ratio preferably used in humans by replacing codons encoding amino acids of the FLP protein in a nucleotide sequence encoding a yeast-derived FLP.

In accordance with the present invention, "codon ratio preferably used in yeast" refers to the frequency of using each codon (codon usage) in the yeast gene among a plurality of codons encoding amino acids, and the values have been disclosed in such a reference as Wada K. et al., Nucleic Acids Res. 18(Suppl.): 2367-2411, 1990. Specific examples thereof are shown in Table 1. "Standard values for the yeast gene" in Table 1 represents the frequency of using each codon for each amino acid in genes of a yeast (S. cerevisiae) described in the above reference. Similarly, "codon ratio preferably used in humans" represents the frequency of using each codon for each amino acid in human genes, and specific values thereof are shown in "Standard values for human genes."

In accordance with the present invention, "codon ratio preferably used in yeast" may be simply designated "yeast type codon" and "codon ratio preferably used in humans" may be simply designated "human type codon."

In accordance with the present invention, "a codon ratio preferably used in yeast is changed to a codon ratio preferably used in humans" means that the frequency of using each codon is brought closer to "the standard values for human genes" in Table 1 by replacing the codon used without changing the amino acid sequence of a protein. The ratio that is brought closer to "the standard values for human genes" is not limited, as long as it satisfies the requirement of enhancing the translation efficiency of the FLP protein in animal cells including human cells by changing to human type codons.

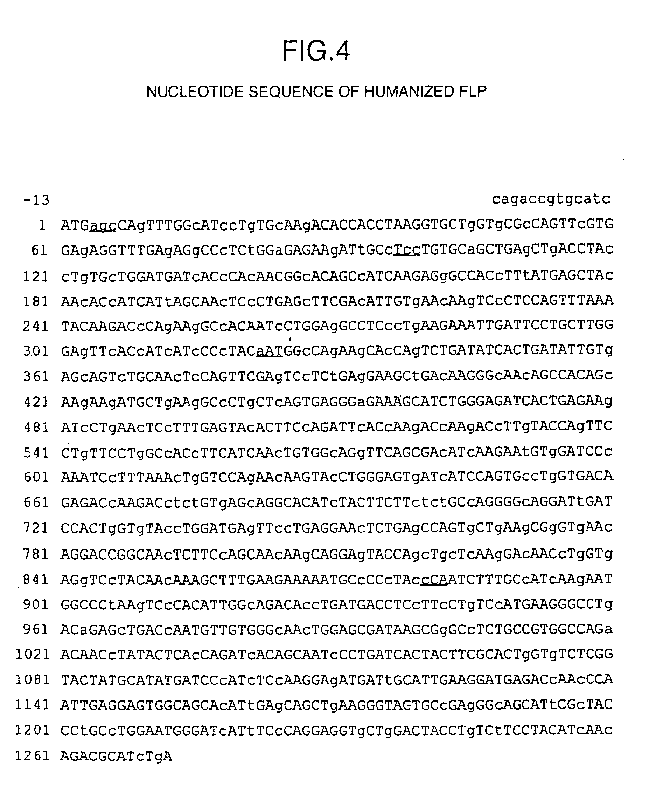

In accordance with the present invention, an example of the nucleotide sequence (SEQ ID NO: 5) of FLP changed to "human type codons" is shown in Figure 4 and Table 1. In Table 1 and the present specification, "yeast type FLP" represents the FLP that has a nucleotide sequence inherent to yeast, and "humanized FLP" represents the FLP that has a nucleotide sequence changed to "human type codons." With reference to "yeast type FLP" and "humanized FLP" shown in Table 1, by way example, replacement from "a yeast type codon" to "a human type codon" is explained in further details.

For example, two codons "TGT" and "TGC" are used for cysteine. As can be seen in Table 1, there are five cysteines in FLP and the yeast type FLP uses only the "TGT" codon. By changing three out of five "TGT" codons to the "TGC" codons, the ratio of "TGT" codons becomes 40% which is approximately equal to "the standard values for human genes." In this way, bringing the codons of amino acids of the entire FLP protein closer to "the standard values for human genes" means replacement to "the human type codon" of the present invention.

However, in stead of mechanically replacing codons to bring closer to "the standard values for human genes," contrivances are included in the present invention in that, for example, when the same amino acid occur contiguously, the same codon is not used and the codon of a second amino acid or after is changed so that the same codon does not occur contiguously.

In the FLP changed to "the human type codon" of the present invention, the 5'-end is preferably in accordance with the Kozak sequence. The Kozak sequence represents nucleotide sequences frequently existed around the translation initiation point of vertebrate genes, of which details are disclosed in the literature (Kozak M., Nucleic Acids Res. 9: 5233-5262, 1981; Kozak M., J. Cell Biol. 108: 229-241, 1989, and the like).

The FLP changed to "the human type codon" may further contain amino acid substitution for increasing its enzymatic activity at 37°C. It is known that the optimum temperature of FLP enzymatic activity is 30°C, and thus at ordinary temperature (37°C) for culturing animal cells the enzymatic activity of FLP decreases (Buchholz F. et al., Nucleic Acids Res. 24: 4256-4262, 1996). As a contrivance to enhance the enzymatic activity of the FLP protein at 37°C, it is reported, 4 amino acids of the FLP protein were replaced with other amino acids resulting in enhanced activity (Buchholz F. et al., Nat. Biotechnol. 16: 657-662, 1998). More specifically, a second amino acid of the FLP amino acid sequence was substituted from proline to serine, a 33rd amino acid from leucine to serine, a 108th amino acid from tyrosine to asparagine, and a 294th amino acid from serine to proline. The example of humanized FLP of the present invention shown in Table 1 and Figure 4 contains replacement of these four amino acids.

Subsequently, by way of example, a method of preparing DNA containing the FLP having a nucleotide sequence changed to the human type codon of the present invention is described for the humanized FLP having a nucleotide sequence as shown in Figure 4. Sense and antisense strands of DNA comprising 30-40 bases corresponding to the nucleotide sequence shown in Figure 4 are synthesized. At this time, the nucleotide sequences of both strands are desined to overlapping. Furthermore, in order to clone into plasmid, a PstI site is added to the 5'-end and a KpnI site is added to the 3'-end. Since there is one site each for EcoRV and HindIII in the humanized FLP gene, DNAs corresponding to the PstI-EcoRV fragment, the EcoRV-HindIII fragment, and the HindIII-KpnI fragment are each annealed and then are separately cloned into plasmids, and then these fragments are ligated to construct a plasmid containing the full-length FLP.

After DNA thus prepared containing the humanized FLP gene is ligated to downstream of a suitable promoter, that can be expressed in animal cells, the DNA may be transduced in the form of plasmid DNA to a human or animal cell, or may be transduced by a viral vector preferably an adenovirus vector.

Methods of confirming enhancement in the amount expressed of the FLP protein transfected into a human or animal cell include, but not limited to, a method of determining the amount of the FLP protein per se, a method of determining the function of the FLP protein, and the like: As a method of determining the amount expressed of the FLP protein per se, there can be mentioned a Western blot method using anti-FLP antibody and the like. As a method of determining the function of the FLP protein, there can be used a method of determining the efficiency of recombination using the DNA containing the FRT sequence as substrate, the cell-disrupted solution containing the FLP protein, as enzymatic solution, a method measuring of the manifestation of some property or other of introducing the substrate DNA simultaneously with the FLP gene into the cell, resulting recombination of the substrate DNA. As an example of the latter case, there can be mentioned a method of using the substrate DNA having a structure of a promoter/FRT sequence/neomycin resistant gene/poly(A) sequence/FRT sequence/lacZ gene/poly(A) sequence, and then determining the activity of the gene product of lacZ. β-galactosidase, and the like.

**Table 1**

| AA | Codon | Yeast-type FLP | | Yeast gene standard value (%) | Humanized FLP | | Human-gene standard value (%) |
|---|---|---|---|---|---|---|---|
| | | No. of codons | % | | No. of codons | % | |
| End | TAA | 1 | 100.0 | 52.4 | 0 | 0.0 | 29.2 |
| | TAG | 0 | 0.0 | 19.0 | 0 | 0.0 | 20.8 |
| | TGA | 0 | 0.0 | 28.6 | 1 | 100.0 | 50.0 |
| Ala | GCT | 8 | 33.3 | 44.1 | 4 | 15.7 | 28.0 |
| | GCC | 4 | 16.7 | 24.1 | 15 | 62.5 | 41.6 |
| | GCG | 1 | 4.2 | 7.9 | 0 | 0.0 | 10.3 |
| | GCA | 11 | 45.8 | 23.8 | 5 | 20.8 | 20.0 |
| Cys | TGT | 5 | 100.0 | 67.3 | 2 | 40.0 | 40.6 |
| | TGC | 0 | 0.0 | 32.7 | 3 | 60.0 | 59.4 |
| Asp | GAT | 13 | 92.9 | 62.4 | 9 | 64.3 | 42.8 |
| | GAC | 1 | 7.1 | 37.6 | 5 | 35.7 | 57.2 |
| Glu | GAG | 11 | 42.3 | 25.7 | 24 | 92.3 | 60.7 |
| | GAA | 15 | 57.7 | 74.3 | 2 | 7.7 | 39.3 |
| Glu | GAG | 11 | 42.3 | 25.7 | 24 | 92.3 | 60.7 |
| | GAA | 15 | 57.7 | 74.3 | 2 | 7.7 | 39.3 |
| Phe | TTC | 9 | 42.9 | 46.3 | 14 | 66.7 | 58.9 |
| | TTT | 12 | 57.1 | 53.7 | 7 | 33.3 | 41.1 |
| Gly | GGC | 3 | 17.6 | 15.4 | 12 | 70.6 | 35.8 |
| | GGA | 8 | 47.1 | 15.4 | 3 | 17.6 | 24.1 |
| | GGG | 1 | 5.9 | 8.8 | 1 | 5.9 | 24.4 |
| | GGT | 5 | 29.4 | 60.4 | 1 | 5.9 | 15.8 |
| His | CAC | 4 | 44.4 | 40.0 | 9 | 100.0 | 60.4 |
| | CAT | 5 | 55.6 | 60.0 | 0 | 0.0 | 39.6 |
| I1E | ATC | 9 | 25.0 | 29.5 | 24 | 66.7 | 54.0 |
| | ATA | 17 | 47.2 | 20.5 | 0 | 0.0 | 12.9 |
| | ATT | 10 | 27.8 | 49.9 | 12 | 33.3 | 33.1 |
| Lys | AAA | 22 | 61.1 | 51.7 | 6 | 16.7 | 38.9 |
| | AAG | 14 | 38.9 | 48.3 | 30 | 83.3 | 61.1 |
| Leu | CTT | 6 | 15.4 | 10.6 | 0 | 0.0 | 11.2 |
| | CTA | 9 | 23.1 | 13.1 | 0 | 0.0 | 6.5 |
| | CTG | 3 | 7.7 | 9.2 | 30 | 78.9 | 44.5 |
| | TTG | 8 | 20.5 | 35.5 | 4 | 10.5 | 11.5 |
| | TTA | 11 | 28.2 | 27.1 | 0 | 0.0 | 5.5 |
| | CTC | 2 | 5.1 | 4.5 | 4 | 10.5 | 20.8 |
| Met | ATG | 7 | 100.0 | 100.0 | 7 | 100.0 | 100.0 |
| Asn | AAC | 3 | 13.6 | 45.1 | 17 | 73.9 | 57.7 |
| | AAT | 19 | 86.4 | 54.9 | 6 | 26.1 | 42.3 |
| Pro | CCC | 1 | 6.7 | 13.3 | 5 | 33.3 | 35.3 |
| | CCT | 6 | 40.0 | 29.0 | 5 | 33.3 | 27.3 |
| | CCA | 7 | 46.7 | 48.4 | 5 | 33.3 | 25.7 |
| | CCG | 1 | 6.7 | 9.3 | 0 | 0.0 | 11.6 |
| Gln | CAG | 9 | 52.9 | 25.9 | 16 | 94.1 | 75.2 |
| | CAA | 8 | 47.1 | 74.1 | 1 | 5.9 | 24.8 |
| Arg | GGT | 2 | 10.0 | 16.9 | 0 | 0.0 | 8.9 |
| | AGA | 7 | 35.0 | 54.1 | 4 | 20.0 | 18.8 |
| | CGC | 1 | 5.0 | 4.5 | 3 | 15.0 | 21.4 |
| | CGA | 2 | 10.0 | 5.2 | 0 | 0.0 | 10.2 |
| | AGG | 7 | 35.0 | 16.9 | 10 | 50.0 | 21.0 |
| | CGG | 1 | 5.0 | 2.5 | 3 | 15.0 | 19.7 |
| Ser | TCA | 12 | 29.3 | 19.5 | 0 | 0.0 | 12.8 |
| | TCC | 1 | 2.4 | 18.0 | 16 | 38.1 | 24.4 |
| | TCG | 5 | 12.2 | 8.1 | 0 | 0.0 | 5.8 |
| | TCT | 6 | 14.6 | 30.8 | 10 | 23.8 | 18.2 |
| | AGC | 10 | 24.4 | 8.9 | 13 | 31.0 | 25.8 |
| | AGT | 7 | 17.1 | 14.6 | 3 | 7.1 | 13.0 |
| Thr | ACG | 3 | 10.7 | 11.5 | 0 | 0.0 | 11.8 |
| | ACC | 3 | 10.7 | 23.9 | 17 | 60.7 | 40.5 |
| | ACT | 9 | 32.1 | 37.8 | 4 | 14.3 | 22.4 |
| | ACA | 13 | 46.4 | 26.8 | 7 | 25.0 | 25.4 |
| Val | GTC | 4 | 21.1 | 24.4 | 1 | 5.3 | 25.7 |
| | GTG | 5 | 26.3 | 15.6 | 17 | 89.5 | 48.7 |
| | GTT | 5 | 26.3 | 43.6 | 1 | 5.3 | 16.4 |
| | GTA | 5 | 26.3 | 16.4 | 0 | 0.0 | 9.3 |
| Trp | TGG | 6 | 100.0 | 100.0 | 6 | 100.0 | 100.0 |
| Tyr | TAC | 11 | 52.4 | 49.8 | 17 | 85.0 | 60.1 |
| | TAT | 10 | 47.6 | 50.2 | 3 | 15.0 | 39.9 |

The present invention will now be explained in more details hereinbelow with reference to Examples. It should be noted, however, that the present invention is not limited by these examples in any way and that variations and modifications known in the filed of art can be made. Unless otherwise note, procedures for handling phages, plasmids, DNA, various enzymes, Escherichia coli, culture media etc. were performed as described in "Molecular Cloning, A Laboratory Manual, edited by T. Maniatis, et al., Second edition, 1989, Cold Spring Harbor Laboratory."

Cosmid vectors used in the Examples, pAxCAwt (Kanegae Y. et al., Nucleic Acids Res. 23: 3816-3821, 1995) and pAxcw (Japanese Unexamined Patent Publication (Kokai) No. 8-308585, page 15, pAdexlcw and pAxcw are identical), are vectors that contain the majority of adenovirus type 5 genome other than adenovirus E1 and E3 genes. pAxCAwt has introduced the CAG promoter into the E1 gene deletion site and has a cloning site in between the promoter and the poly(A) sequence. pAxcw has only inserted ClaI and SwaI sites in the E1 gene deletion site.

### Example 1

### Construction of a cell line (293FNCre cells) expressing recombinase Cre in a FLP protein-dependent manner

In order to obtain a cell line that has inserted DNA having a structure CAG promoter/FRT sequence/neomycin resistant gene/SV40 poly(A) sequence/FRT sequence/nuclear localization signal-tagged Cre gene/β-globin poly(A) sequence on the chromosome of an animal cell, the following procedure was followed.

A synthetic DNA (SEQ ID NO: 1) composing of 54 bases containing a 34 bp FRT sequence and a complementary strand thereof were inserted into a SmaI site of plasmid pUC18 to obtain a plasmid pUFwF (2.8 kb) having two FRT sequences in the same direction and a SwaI site in between the two FRT sequences.

A fragment containing a neomycin resistant gene and SV40 poly(A) sequence that was obtained by digesting plasmid pCALNLZ (Y. Kanegae et al., Gene 181: 207-212, 1996, Figure 1A) with MluI and XhoI and then blunt-ending was inserted into the SwaI site of pUFwF to obtain a plasmid pUFNF (Figure 1B).

A 27 bases synthetic polylinker (5'-AAA TTG AAT TCG AGC TCG GTA CCC GGG-3', SEQ ID NO: 2) and a complementary strand thereof were inserted into the SwaI site of plasmid pCALNLw (Y. Kanegae et al., Gene 181: 207-212, 1996) to obtain a plasmid pCALNL5 (Figure 1C). An about 1.2 kb fragment containing FRT sequence/neomycin resistant gene/SV40 poly(A) sequence/FRT sequence obtained by digesting pUFNF with BamHI and Asp718 and then blunt-ending, was ligated to an about 4.9 kb fragment containing the CAG promoter obtained by digesting pCALNL5 with MluI and XhoI and then blunt-ending, to obtain a plasmid pCAFNF5 (6.1 kb).

An about 1.2 kb fragment containing a nuclear localization signal-added Cre gene (NCre) that was obtained by digesting plasmid pSRNCre (Y. Kanegae et al., Nucleic Acids Res. 23: 3816-3821, 1995) with PstI and XbaI and then blunt-ending was inserted into the SwaI site of pCAFNF5 to obtain a plasmid pCAFNFNCre (Figure 2).

The 293 cells were transfected (calcium phosphate coprecipitation method) with pCAFNFNCre and then G418 (neomycin derivative) resistance cells were single-cloned to obtain a plurality of cell lines (293FNCre cells).

### Example 2

### Construction of recombinase FLP-expressing plasmid and recombinant adenovirus

In order to obtain a plasmid in which the nucleotide sequences around the translation initiation codon of the recombinase FLP were in accordance with the Kozak sequence, the following procedure was followed:
(a) Plasmid ·pUCFLP is a plasmid in which a fragment (1457 bp) containing the full-length FLP gene from the SphI site (position 5568) to the XbaI site (position 703) of 2 micron DNA (6318 bp: James et al., Nature 286: 860-865, 1980) of yeast has been inserted to the SphI-XbaI site of plasmid pUC19. pUCFLP was digested with XbaI and SphI to obtain an about 1.5 kb fragment containing the full-length FLP gene.
(b) A synthetic DNA adaptor with the following sequences in which the 5'-end overhang can be ligated to a HindIII cleavage site and the other end can be ligated to a SphI cleavage site and which has a PstI site upstream of the translation initiation codon was synthesized.
5'-AG CTT CTG CAG CAG ACC GTG CAT CAT G-3' (SEQ ID NO: 3)
3'-A GAC GTC GTC TGG CAC GTA-5' (SEQ ID NO: 4)

Both DNAs in (a) and (b) were inserted into the HindIII-XbaI site of pUC19 to obtain a plasmid pUKFLP (4.1 kb).

A 1.4 kb fragment containing the FLP coding region obtained by digesting pUKFLP with PstI and FspI and then blunt-ending was inserted into a SwaI site in between the promoter and the poly(A) sequence of a cosmid vector pAxCAwt to obtain a cosmid vector pAxCAFLP.

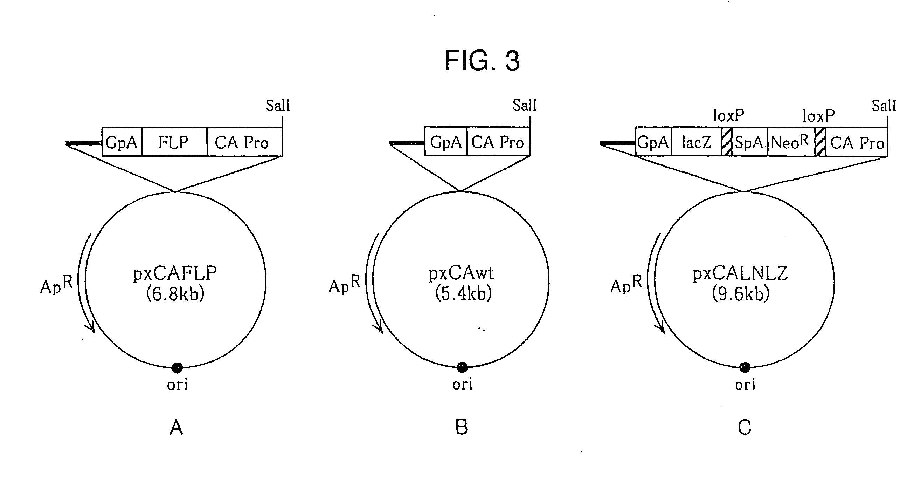

After digesting the above pAxCAFLP with SalI, it was allowed to self-ligate to obtain a FLP expression plasmid pxCAFLP (Figure 3A) in which the majority of adenovirus DNA has been deleted (containing about 0.4 kb on the left end). Similarly, a plasmid pxCAwt (Figure 3B) in which pxCAwt after digesting with SalI was subjected to self-ligation was also constructed. pxCAwt is used as a negative control plasmid for pxCAFLP in Example 3.

293 cells were transfected by the calcium phosphate coprecipitation method according to a known method (Miyake et al., Proc. Natl. Acad. Sci. 93: 1320-1324, 1996) with pAxCAFLP and an adenovirus DNA-terminal protein complex to obtain the FLP-expressing recombinant adenovirus of interest, AxCAFLP (E1 and E3 genes are deleted).

### Example 3

### Confirmation of expression of the Cre protein depending on the FLP protein of the 293FNCre cells

### (1) Construction of a plasmid that expresses the lacZ gene depending on the Cre protein

A cosmid vector pAxCALNLZ (Kanegae et al., Nucleic Acids Res. 23: 3816-3821, 1995) is a cosmid in which a CAG promoter/loxP sequence/neomycin resistant gene/SV40 poly(A) sequence/loxP sequence/E. coli lacZ gene/β-globin poly(A) sequence has been inserted into the E1 gene deletion site of the cosmid vector pAxcw. After digesting pAxCALNLZ with SalI, it was allowed to self-ligate thereby to construct a plasmid pxCALNLZ (Figure 3C) in which the majority of adenovirus DNA has been removed (containing about 0.4 kb on the left end). pxCALNLZ is a vector that expresses the lacZ gene depending on the Cre protein.

### (2) Expression of the Cre protein depending on the FLP protein of 293FNCre cells

The expression of the Cre protein by 293FNCre cells in a FLP protein-dependent manner was confirmed by the cotransfection method of the plasmid pxCAFLP and the plasmid pxCALNLZ constructed in Example 2. This is based on the principle that the FLP protein expressed by pxCAFLP acts on the chromosome of the 293FNCre cells and then excise a stuffer sequence (the neomycin resistant gene and the SV40 poly(A) sequence) between two FRT sequences resulting in the expression of the Cre protein from the CAG promoter. The expressed Cre protein excises a stuffer sequence (the neomycin resistant gene and the SV40 poly(A) sequence) between two loxP sequences of the plasmid pxCALNLZ, resulting in the expression of the lacZ gene. Thus, the expression of the lacZ gene by the cell in which these two plasmids were cotransfected should provide evidence that the 293FNCre cells expressed the Cre protein in a FLP protein-dependent manner. Details of the experimental method and the results are shown below.

Out of the 293FNCre cell clones obtained in Example 1, six clones (#1, #2, #3, #6, #8, and #9) were cultured in a 6-well plate and cells were cotransfected with 0.5 µg of pxCAFLP and 0.5 µg of pxCALNLZ by the calcium phosphate coprecipitation method. As a negative control, pxCAwt constructed in Example 2 was used in stead of pxCAFLP.

Three days later, after the culture liquid was removed and the cell surface was washed with PBS(-), 0.25% glutaraldehyde solution was added, the cells were fixed at 4°C for 10 minutes, and then washed again with PBS(-). In order to identify the expressed β-galactosidase, a X-Gal staining solution (5 mM potassium ferricyanide/5 mM potassium ferrocyanide/2 mM magnesium chloride/1 mg/ml X-Gal (5-bromo-4-chloro-3-indolyl-β-D-galactoside)/PBS(-)) was added followed by staining for 5 hours.

As a result of the above experiment, in four clones #1, #3, #6, and #9, when cotransfected with pxCAwt and pxCALNLZ the ratio of the blue stained cells by expressing β-galactosidase were a few percent or less, whereas when cells were cotransfected with pxCAFLP and pxCALNLZ about 50% of the cells were stained blue. Thus, it was demonstrated that in these four clones, a -FRT sequence/neomycin resistant gene/SV40 poly(A) sequence/FRT sequence- was correctly inserted in between the promoter and the Cre gene and that they express the Cre protein at a high level in a FLP protein-dependent manner.

### Industrial Applicability

The present invention provides cells useful for the production of recombinant viral vectors, specifically recombinant adenovirus vectors. The present invention permits efficient production of recombinant viral vectors, and facilitates the supply of recombinant viral vectors available in the field of gene therapy.

### Sequence listing free text

The nucleotide sequence as set forth in SEQ ID NO: 1 is a recognition sequence of FLP.

The nucleotide sequence as set forth in SEQ ID NO: 2 is a polylinker.

The nucleotide sequence as set forth in SEQ ID NO: 3 is a sense strand of the adapter.

The nucleotide sequence as set forth in SEQ ID NO: 4 is an antisense strand of the adapter.

The nucleotide sequence as set forth in SEQ ID NO: 5 is the entire sequence of humanized FLP.

### ADDITIONAL ASPECTS OF THE INVENTION

1. A cell that expresses recombinase Cre in the presence of recombinase FLP in a FLP-dependent manner.
2. The cell according to aspect 1 that expresses the adenovirus E1A gene.
3. The cell according to aspect 1 or 2 that derives from human fetus kidney-derived cell line 293 cells.
4. The cell according to any of aspects 1-3 having, in the genome thereof, a promoter, a recognition sequence of recombinase FLP, a stuffer sequence, a recognition sequence of recombinase FLP, and the recombinase Cre gene sequence in this order from upstream.
5. The cell according to aspect 4, wherein the promoter is a hybrid promoter (CAG promoter) comprising a cytomegalovirus enhancer, a chicken β-actin promoter, a splicing acceptor and poly(A) sequence of rabbit β-globin.
6. The cell according to aspect 4 or 5 wherein the stuffer sequence comprises a nucleotide sequence that acts so as to suppress the expression of the Cre gene located downstream thereof.
7. The cell according to aspect 6 which comprises a poly(A) sequence, or a nucleotide sequence encoding the desired protein and a poly(A) sequence, as a nucleotide sequence that acts so as to suppress the expression of the Cre gene.
8. The cell according to aspect 7 wherein the desired protein is the product of a drug resistant gene.
9. The cell according to aspect 8 wherein the drug resistant gene is a neomycin resistant gene.
10. The cell according to any of aspects 4-9 having a nuclear localization signal at the 5'-end or 3'-end of the recombinase Cre gene.
11. A method of expressing recombinase Cre by introducing recombinase FLP into the cell according to any of aspects 4-10.
12. The method according to aspect 11 wherein the method of introducing recombinase FLP uses an adenovirus vector.
13. A method of producing a recombinant viral vector which comprises using a cell that expresses recombinase Cre in the presence of recombinase FLP in a FLP-dependent manner.
14. A method of producing a recombinant adenovirus vector using the method according to aspect 11 or 12 and aspect 13.
15. The method according to aspect 14 characterized in that the adenovirus vector has, in the genome thereof, a recognition sequence of recombinase Cre, an adenovirus packaging sequence, and a recognition sequence of recombinase Cre in this order from upstream.

## Claims

1. DNA having a modified nucleotide sequence wherein the translation efficiency of the FLP protein in animal cells including human cells has been enhanced by changing a codon ratio preferably used in yeast to a codon ratio preferably used in humans by replacing a codon encoding an amino acid of the FLP protein in a nucleotide sequence encoding a yeast-derived FLP.

2. The DNA according to claim 1 wherein the 5'-end region of the nucleotide sequence encoding FLP is in accordance with the Kozak sequence.

3. The DNA according to claim 1 or 2 wherein the translation efficiency of the FLP protein at 37°C has been enhanced.

4. The DNA according to any one of the preceding claims wherein the second amino acid is serine, 33^{rd} amino acid is serine, 108^{th} amino acid is asparagine, and 294^{th} amino acid is proline in the amino acid sequence of FLP.

5. The DNA according to claim 4 which is the nucleotide sequence as set forth in SEQ ID NO: 5.

6. A plasmid or viral vector comprising the DNA of any one of the preceding claims.

7. A human or animal cell comprising the DNA of any one of claims 1 to 5.
